# EUROPÄISCHE PATENTSCHRIFT

(11) **EP 1 159 444 B1**
(45) Veröffentlichungstag und Bekanntmachung des Hinweises auf die Patenterteilung: **26.05.2004**
(21) Anmeldenummer: 00916893.1
(22) Anmeldetag: 02.03.2000
(51) Int. Cl.: C12Q 1/02, C12M 3/06, B01J 19/00

(54) **MEMBRANMODUL ZUM TESTEN VON WIRKSTOFFEN AN ZELLEN**
MEMBRANE MODULE FOR TESTING ACTIVE SUBSTANCES AT CELLS
MODULE A MEMBRANES DESTINE A TESTER DES PRINCIPES ACTIFS SUR DES CELLULES

(30) Priorität: 09.03.1999 DE 19910539
(43) Veröffentlichungstag der Anmeldung: 05.12.2001
(73) Patentinhaber: Acordis Industrial Fibers GmbH, 42103 Wuppertal (DE)
(72) Erfinder: GLÖCKNER, Herma, D-63839 Kleinwallstadt (DE); LEMKE, Horst-Dieter, D-63785 Obernburg (DE); HAUCK, Friedrich, D-63920 Grossheubach (DE); ZIMMERER, Christoph, D-63916 Amorbach (DE); WOLLBECK, Rudi, D-63906 Erlenbach (DE)
(74) Vertreter: Fett, Günter
(86) Internationale Anmeldenummer: PCT/EP2000/001819
(87) Internationale Veröffentlichungsnummer: WO 2000/053796

(56) Entgegenhaltungen:
- WO-A-99/28438
- DE-C- 19 810 901
- US-A- 4 937 196

## Beschreibung

Die Erfindung betrifft einen Membranmodul zum Testen von Wirkstoffen an Zellen gemäß Oberbegriff des Anspruchs 1 sowie dessen Verwendung.

Im Bereich beispielsweise der Krebsforschung oder Krebstherapie stellt sich vielfach die Aufgabe, zur Beurteilung des möglichen Ausgangs einer Chemotherapie durch schnelle Tests die Wirkung von Cytostatika auf Krebs- oder Tumorzellen eines Krebspatienten zu untersuchen. Hierzu ist es erforderlich, ein Wirkstoff-Screening vorzunehmen, d.h. die Wirkung einer Mehrzahl von Wirkstoffen in verschiedener Dosierung über der Zeit, d.h. mit unterschiedlicher Pharmakokinetik auf die Krebszellen festzustellen und damit das Ansprechen von Patienten auf eine bestimmte Chemotherapie mit größerer Sicherheit vorherzusagen.

Eine solche patientenspezifische Testung bekannter Wirkstoffe unterscheidet sich von den Wirkstofftests, die in sogenannten Tiermodellen durchgeführt werden, bei denen beispielsweise entsprechend der in der WO 94/25074 und in der US 5 676 924 vorgeschlagenen Methode humane Tumorzellen in Hohlfasermembranstücken enkapsuliert werden und diese einem Empfängertier implantiert werden. Zwar wird bei dieser Methode im Unterschied zur direkten Implantierung solcher Zellen in ein Empfängertier durch die Enkapsulierung eine Immunantwort des Empfängertieres verhindert, und es können pro Behandlung des Empfängertiers mit einem Cytostatika unterschiedliche Tumorzellen gleichzeitig untersucht werden. Jedoch hat diese Vorgehensweise ihre Grenzen aufgrund ihrer naturgemäß schlechten Automatisierungsmöglichkeit und der oftmals schlechten Übertragbarkeit der Testergebnisse auf den Menschen.

Daher wird versucht, durch in vitro Untersuchungen zu patientenspezifischen und tumorspezifischen Aussagen zu gelangen. Bei derartigen Untersuchungen wird an Kulturen von Zellen des zu untersuchenden Tumors, d.h. an Zellkulturessays die Wirkung einer Testsubstanz, d.h. eines Wirkstoffes auf die Zellkultur untersucht. Bei derartigen Untersuchungen werden üblicherweise Standardkulturgefäße und methoden eingesetzt, die jedoch ein dreidimensionales Wachstum der Zellen nicht zulassen, d.h. reale Tumore können nicht simuliert werden. Die Wirksamkeit z.B. von Cytostatika wird aber entscheidend durch die räumliche Anordnung der Zellen und deren Zugänglichkeit beeinflusst. Darüber ist die Nachstellung einer Pharmakokinetik, d.h. der Einfluss eines bestimmten Konzentrations-Zeit-Verlaufs des Wirkstoffes oder einer Kombinationstherapie, bei der verschiedene Wirkstoffe nacheinander zeitlich versetzt untersucht werden sollen, mit den bekannten in vitro Methoden nicht oder nur sehr eingeschränkt möglich.

In der WO-A-0 053 797 wird ein Verfahren zum in vitro Testen von Wirkstoffen unter Verwendung eines Membranmoduls vorgeschlagen, mittels dessen die Nachteile der bekannten in vitro Tests zumindest reduziert werden. Hierbei werden z.B. Tumorzellen im Zellkulturraum des Membranmoduls vorgelegt, wobei im Zellkulturraum Membransysteme zur Zuführung eine Nährmediums und zur Oxygenation, d.h. zur Versorgung der Zellen mit Sauerstoff angeordnet sind, und es wird mit einem definierten Konzentrations-Zeit-Verlauf mindestens ein zu untersuchender Wirkstoff in den Zellkulturraum zudosiert, wobei gleichzeitig die Zellvitalität der im Zellkulturraum befindlichen Zellen überwacht wird.

Es sind verschiedene Membranmodule zur Züchtung von Zellen bekannt. So wird in der DE-A-36 33 891 eine Vorrichtung zur Kultivierung von tierischen Zellen beschrieben, mittels dessen Zellen vermehrt und Wertstoffe aus den Zellen, d.h. Produkte gewonnen werden sollen. Diese Vorrichtung enthält in mattenförmiger Anordnung zueinander im wesentlichen parallele Kapillarmembranen, deren Enden in Vergussmassen eingebettet sind und über die eine Versorgung der Zellen mit Nährstoffen erfolgt. In dieser Vorrichtung können auch weitere Membranen zur verbesserten Sauerstoffversorgung der Zellen eingesetzt werden. Im fertigen Membranmodul liegen die Kapillarmembranen im wesentlichen parallel zueinander und zur Gehäuseachse.

Die US 5 516 691 offenbart einen Modul zur Züchtung und zur Nutzung der Stoffwechsetleistung zum Erhalt von Mikroorganismen, insbesondere von Zellen oder Bakterien. Der Modul besteht aus einem Außengehäuse, mindestens drei unabhängigen Membransystemen, wobei mindestens zwei Membransysteme als Hohlfasermembranen ausgebildet sind und diese Hohlfasermembranen ein dichtgepacktes Netzwerk beispielsweise aus in überkreuzter Anordnung übereinandergelegten Schichten dieser Hohlfasermembranen ausbilden. Die Hohlfasermembranen sind an mindestens einem ihrer Enden in Vergussmassen eingebettet und kommunizieren mit mindestens einem Einlass bzw. einem Einlass und einem Ausiass. Im Außenraum zwischen den Hohlfasermembranen befinden sich Mikroorganismen, insbesondere Zellen. Ober die verschiedenen Hohlfasermembransysteme erfolgt der Transport von Nährstoffen, Gasen und Stoffwechselprodukten zu bzw. von den sich im Innenraum des Moduls befindlichen Zellen.

Der Membranmodul gemäß der US 5 516 691 wird vorzugsweise zur Züchtung von Leberzellen verwendet und in einem extrakorporalen Leberunterstützungssystem eingesetzt. Hierbei kommt es darauf an, die Stoffwechselleistung der Zellen zu nutzen, d.h. es muss eine möglichst große Zahl von Zellen im Membranmodul gezüchtet und somit ein großes Modulvolumen zur Ausnahme der Zellen bereitgestellt werden. Hierzu wird in der US 5 516 691 ein Versuchsmodul mit den Außenabmessungen 12x12 cm beschrieben, bei dem 100 Schichten von Hohlfasermembranen übereinander angeordnet sind und weitere 50 Hohlfasermembranschichten vertikal von oben nach unten zur Ausbildung eines dichtgepackten Netzwerkes mit den anderen Schichten verwoben sind. Die offenbarten Membranmodule weisen einen komplexen Aufbau auf und sind für ein Wirkstoffscreening, bei dem eine Vielzahl von Membranmodulen zum Einsatz gelangen muss, nicht geeignet.

Weder die in der DE-A-36 33 891 noch die in der US 5 516 691 offenbarten Modulkonstruktionen sind für das Testen von Wirkstoffen auf Zellen und insbesondere für ein Wirkstoffscreening einsetzbar.

Es ist daher Aufgabe der vorliegenden Erfindung, einen Membranmodul zur Verfügung zu stellen, der zum Testen von Wirkstoffen an Zellen geeignet ist und insbesondere sich bei einem Screening solcher Wirkstoffe, d.h. bei der Testung einer Vielzahl von Wirkstoffen, die darüber hinaus jeweils mit verschiedenen Konzentrations-Zeit-Verläufen zu untersuchen sind, verwenden lässt.

Die Aufgabe wird gelöst durch einen Membranmodul zum Testen von Wirkstoffen an Zellen, umfassend ein Gehäuse mit einem Innenraum, welcher durch einen Deckel, einen Boden sowie eine Seitenwand begrenzt wird, im Innenraum angeordnet ein System von ersten Kapillarmembranen und ein System von zweiten Kapillarmembranen und gegebenenfalls mindestens ein weiteres System von Kapillarmembranen, wobei jede Kapillarmembran jeweils ein erstes und ein zweites Ende sowie ein mit einem Fluid beschickbares Lumen aufweist, wobei die Kapillarmembranen im Innenraum zu mindestens einer zum Boden parallelen flächigen Schicht angeordnet sind und im Innenraum des Gehäuses im extrakapillären Raum um die Kapillarmembranen herum ein Zellkulturraum ausgebildet ist, wobei die Kapillarmembranen mit mindestens einem ihrer Enden durch die Seitenwand des Innenraums hindurchtreten und so nach Systemen getrennt an diesem mindestens einen Ende jeweils in einer Vergussmasse eingebettet sind, dass der Innenraum fluiddicht nach außen abgedichtet ist, und wobei die Kapillarmembranen eines jeden Systems mit ihrem Lumen mit einer Einlasseinrichtung und/oder einer Auslasseinrichtung in Fluidverbindung stehen, wobei der Membranmodul dadurch gekennzeichnet ist, dass der Innenraum ein Volumen zwischen 0,1 und 5 cm³ aufweist.

Durch die erfindungsgemäße Ausgestaltung des Membranmoduls wird es zum einen möglich, die Zellen, an denen die Wirkstoffe getestet werden sollen, unter Kulturbedingungen zu halten, die die in vivo Bedingungen, d.h. die Bedingungen im lebenden Organismus möglichst genau simulieren. Zum anderen erlaubt das erfindungsgemäß kleine Innenraumvolumen des erfindungsgemäßen Membranmoduls zwischen 0,1 und 5 cm³ seine Verwendung zum Testen von Wirkstoffen und insbesondere zum Wirkstoffscreening. Ein solches Wirkstoffscreening ist nur dann möglich, wenn zu dem einzelnen Wirkstofftest nur eine geringe Menge an Zellmaterial verbraucht wird, so dass z.B. einem Krebspatienten nur eine geringe Menge an Zellen entnommen werden muss, um mehrere Wirkstoffe, also z.B. mehrere Cytostatika, oder auch Wirkstoffkombinationen auch mit unterschiedlichem Wirkstoffprofil, d.h. unterschiedlicher Pharmakokinetik auf ihre Eignung zur Krebsbekämpfung zu testen, d.h. also das Wirkstoffscreening durchzuführen. Hierzu wird dann eine Vielzahl der erfindungsgemäßen Membranmodule eingesetzt, die in einem entsprechenden modular gestalteten System parallelgeschaltet werden können. Bevorzugt wird daher der erfindungsgemäße Membranmodul zum Testen von Wirkstoffen an Zellen verwendet. Unter zu testenden Wirkstoffen werden dabei solche Stoffe verstanden, deren Wirkung auf die in Untersuchung befindlichen Zellen vor dem Test oder deren patientenoder tumorspezifische Wirkung nicht oder nicht ausreichend vorhersagbar ist. Derartige Wirkstoffe sind z.B. Cytostatika, Antibiotika, Cytokine, Wachstumsfaktoren oder antivirale Agentien.

Die im erfindungsgemäßen Membranmodul eingesetzten Kapillarmembranen können verschiedenartige äußere Konturen, d.h. im Querschnitt betrachtet verschiedene äußere Umrisse aufweisen. Die Kapillarmembranen können beispielsweise eine im wesentlichen runde bzw. kreisförmige, dreieckige, viereckige, sechseckige oder achteckige Kontur aufweisen, sie können auch oval, elliptisch, dreilappig, vierlappig usw. ausgebildet sein. Es können auch die Kapillarmembranen unterschiedlicher Systeme unterschiedliche äußere Konturen aufweisen. Entsprechendes gilt bezüglich des Innenquerschnitts der Kapillarmembranen. Bevorzugt werden Kapillarmembranen mit im wesentlichen kreisförmiger Kontur eingesetzt.

Zur Vermeidung von Totraumzonen im Innenraum weist der Innenraum des erfindungsgemäßen Moduls in Blickrichtung senkrecht zur mindestens einen Kapillarmembranschicht bevorzugt einen kreisförmigen Innenquerschnitt auf. Besonders bevorzugt hat der Innenraum einen Durchmesser zwischen 10 und 20 mm. In einer besonders bevorzugten Ausführung hat der Innenraum ein Volumen zwischen 0,3 und 3,0 ml.

Vorzugsweise werden die ersten Kapillarmembranen zur kontinuierlichen Zuführung von Nährstoffen und zum Abführen von Stoffwechselprodukten eingesetzt. In diesem Fall sind beide Enden der ersten Kapillarmembranen jeweils in eine Vergussmasse eingebettet und das Lumen der ersten Kapillarmembranen steht sowohl mit einer Einlasseinrichtung als auch mit einer Auslasseinrichtung in Fluidverbindung und ist von der Nährmediumlösung im cross flow Modus durchströmbar. Die ersten Kapillarmembranen müssen zur Zuführung eines flüssigen Nährmediums geeignet sein. Vorzugsweise handelt es sich also um eine Membran, die einen kontinuierlichen Stofftransport durch die Membranwand aufgrund diffusiver oder konvektiver Transportmechanismen erlaubt. Je nach Erfordernis, d.h. je nachdem, ob ein diffusiver oder ein konvektiver Transport des Nährmediums durch die Membran erforderlich ist, kommen daher Nanofiltrations- oder Ultrafiltrationsmembranen oder Mikrofiltrationsmembranen zum Einsatz.

In einer weiteren bevorzugten Ausgestaltung des erfindungsgemäßen Moduls stehen die ersten Kapillarmembranen mit ihrem Lumen nur mit einer Einlasseinrichtung in Fluidverbindung, nicht hingegen mit einer Auslasseinrichtung. Die ersten Kapillarmembranen dienen in diesem Fall allein der Zuführung einer Nährmediumlösung, die dann im dead end Modus durch die ersten Kapillarmembranen strömt, nicht jedoch zum Abtransport von Stoffwechselprodukten. Hierbei können z.B. beide Enden der zweiten Kapillarmembranen in derselben Vergussmasse eingebettet sein, wobei dann die ersten Kapillarmembranen beispielsweise in U-förmigen Schlaufen im Innenraum angeordnet sind.

Bei einer derartigen Ausgestaltung der ersten Kapillarmembranen ist im Innenraum des erfindungsgemäßen Membranmoduls ein System von dritten Kapillarmembranen angeordnet, mittels dessen die Stoffwechselprodukte der Zellen abtransportiert werden können. Vorzugsweise handelt es sich bei den dritten Kapillarmembranen ebenfalls um im dead end Modus durchströmte Membranen, deren Lumen in diesem Fall allein mit einer Auslasseinrichtung, nicht jedoch mit einer Einlasseinrichtung in Fluidverbindung steht. Bei einer derartigen Ausführungsform des erfindungsgemäßen Membranmoduls sind die Wände der ersten und der dritten Kapillarmembranen bevorzugt konvektiv durchströmbar, wobei die ersten und dritten Kapillarmembranen bevorzugt Mikrofiltrationsmembranen sind. In der Anwendung strömt ein das Nährmedium enthaltender Flüssigkeitsstrom über die ersten Kapillarmembranen in den extrakapillären Zellkulturraum ein und durchströmt diesen, wobei Nährstoffe an die Zellen abgegeben werden und Stoffwechselprodukte an den Flüssigkeitsstrom abgegeben werden. Anschließend wird der mit den Stoffwechselprodukten angereicherte Flüssigkeitsstrom über die dritten Kapillarmembranen aus dem Zellkulturraum abgeleitet. Hierzu ist der erfindungsgemäße Membranmodul vorzugsweise druckdicht ausgeführt bis zu einem Innendruck im Innenraum von 500 mbar.

Die zweiten Kapillarmembranen sind vorzugsweise Membranen, die zur Zuführung eines gasförmigen Stoffes in den Innenraum geeignet sind. Es handelt sich also um Membranen für den Gastransfer von z.B. Sauerstoff in den Modulinnenraum oder von Kohlendioxid aus dem Modulinnenraum, d.h. um Oxygenationsmembranen. Für den bevorzugten Fall, dass über die zweiten Kapillarmembranen also sowohl die Zuführung von Sauerstoff als auch der Abtransport von Kohlendioxid erfolgt, stehen die zweiten Kapillarmembranen sowohl mit einer Einlasseinrichtung als auch mit einer Auslasseinrichtung in Fluidverbindung. Es ist jedoch auch möglich, dass über die ersten Kapillarmembranen nur eine Zuführung von z.B. Sauerstoff erfolgt und der Abtransport von z.B. Kohlendioxid mit dem Abtransport von Stoffwechselprodukten über die ersten oder über die dritten Kapillarmembranen erfolgt. In diesem Fall stehen die zweiten Kapillarmembranen mit ihrem Lumen nur mit einer Einlasseinrichtung in Fluidverbindung und werden im dead end Modus durchströmt.

In einer bevorzugten Ausführungsform des erfindungsgemäßen Membranmoduls stehen die Lumina der ersten und der zweiten Kapillarmembranen sowohl mit einer Einlasseinrichtung als auch mit einer Auslasseinrichtung in Verbindung, sind also im cross flow Modus durchströmbar. In diesem Fall treten die ersten und die zweiten Enden der Kapillarmembranen eines Systems zweckmäßigerweise auf gegenüberliegenden Seiten des Innenraums durch die Seitenwand des Innenraums. Natürlich ist es auch möglich, dass die Durchtrittsstellen der Enden der Kapillarmembranen eines Systems unter einem Winkel zueinander angeordnet sind, beispielsweise unter einem rechten Winkel.

Der erfindungsgemäße Modul umfasst Mittel zur Zuführung eines Wirkstoffes in den Zellkulturraum. In einer bevorzugten Ausführungsform sind diese Mittel die ersten Kapillarmembranen. In diesem Fall wird in der Anwendung dieses Membranmoduls der zu untersuchende Wirkstoff in den Nährmediumstrom eindosiert und tritt mit dem Nährmediumstrom in den Zellkulturraum ein. Konzentrations-Zeit-Profile des Wirkstoffes werden dann unter anderem von der Permeabilität der ersten Kapillarmembranen beeinflusst.

In einer weiteren bevorzugten Ausführungsform bestehen die Mittel zur Zuführung eines Wirkstoffes aus mindestens einem Einlass in den Innenraum z.B. in Form mindestens einer Bohrung durch die Seitenwand, an die zur Modulaußenseite hin eine Einlasseinrichtung bzw. eine Dosiereinheit für den Wirkstoff angeschlossen ist. Der Einlass kann auch mindestens ein Septum, d.h. eine Trennwand zwischen dem Innenraum und der äusseren Umgebung des Moduls, sein, welches Teil des Deckels, des Bodens oder der Seitenwand ist, und welches z.B. mit einer Kapillaren durchstoßen werden kann zur Eindosierung des Wirkstoffes, sich aber nach Entfernen der Kapillaren wieder fluiddicht verschließt. Ein solches Septum kann im übrigen auch vorteilhaft zur Zuführung der Zellkultur in den Innenraum oder zur Entnahme von Proben aus dem Innenraum eingesetzt werden. Die Zuführung einer Zellkultur in den Innenraum kann auch über den Deckel erfolgen, der dann zu diesem Zweck abnehmbar ausgeführt ist. Ebenso kann eine Entlüftung des Zellkulturraums, soweit erforderlich, z.B. mittels einer Kapillaren über das Septum oder ebenfalls über einen abnehmbaren Deckel erfolgen. Die Entlüftung kann jedoch z.B. auch über eine hydrophobe poröse Membran erfolgen, die beispielsweise in die Innenwand eingebettet ist und den Innenraum mit der Aussenseite des Moduls verbindet.

Gemäß einer weiteren vorteilhaften Ausgestaltung sind die Mittel zur Zuführung eines Wirkstoffes ein weiteres System von Kapillarmembranen, über die der zu testende Wirkstoff in gleichmäßiger Verteilung in den Innenraum eindosiert werden kann.

In dem erfindungsgemäßen Membranmodul kann auch ein weiteres System von Kapillarmembranen enthalten sein, mittels dessen z.B. die Entnahme von Proben aus dem Zellkulturraum möglich ist.

Um eine gleichmäßige Nährstoffzufuhr und -abfuhr, eine gleichmäßige Oxygenation und, für den Fall, dass die Wirkstoffe über eines der Membransysteme erfolgt, eine gleichmäßige Wirkstoffzufuhr zu gewährleisten, sind die Kapillarmembranen in Blickrichtung senkrecht zu der mindestens einen Schicht innerhalb der Schichten gleichmäßig über den Innenquerschnitt des Innenraums verteilt. Hierbei liegt der bevorzugte maximale horizontale Abstand zwischen benachbarten Kapillarmembranen innerhalb einer Schicht im Bereich zwischen 50 und 500 µm. Es ist ebenfalls von Vorteil, wenn die Kapillarmembranen unterschiedlicher Systeme in eine in Blickrichtung senkrecht zu den Schichten sich überkreuzende Anordnung gebracht sind. Dies kann z.B. dadurch erreicht werden, dass die ersten und die zweiten Kapillarmembranen miteinander zu einem Gewebe verwoben sind. In einer bevorzugten Ausführungsform sind die Kapillarmembranen nach Systemen getrennt in jeweils mindestens einer Schicht angeordnet, wobei die Schichten wiederum so übereinander angeordnet sind, dass sich die Kapillarmembranen unterschiedlicher Systeme in Blickrichtung senkrecht zu den Schichten überkreuzen.

Vorzugsweise enthält der erfindungsgemäße Membranmodul 2 bis 15 Schichten eines jeden Systems von Kapillarmembranen. Die Gesamtzahl der Kapillarmembranschichten im Innenraum beträgt bevorzugt 2 bis 20.

Aufgrund des Sedimentationsverhaltens der bei Anwendung des erfindungsgemäßen Membranmoduls in den Zellkulturraum eingebrachten Zellen sind in einer vorteilhaften Modulausführung im Innenraum mehrere Schichten von Kapillaren angeordnet, die über der Höhe des Innenraums zwischen Deckel und Boden asymmetrisch mit einer dichteren Schichtenfolge in der Nähe des Bodens verteilt sind.

Bei Membranmodulen mit Innenraumvolumina, wie sie die erfindungsgemäßen Membranmodule aufweisen, verbunden mit einer im Vergleich zu großvolumigeren Modulen geringeren Anzahl an Kapillarmembranen, welche im Innenraum vorzugsweise zu Schichten mit gleichmäßiger Verteilung der Kapillarmembranen über den Innenquerschnitt angeordnet sind, gestaltet sich die Einbettung der Enden der Kapillarmembranen sowie ihre Verbindung mit einer Einlass- und/oder Auslasseinrichtung oftmals schwierig. Bei einer bevorzugten Ausführungsform des erfindungsgemäßen Membranmoduls sind die Enden der Kapillarmembranen der einzelnen Systeme jeweils zu Bündeln zusammengefasst und in ein röhrchenförmiges Endstück, beispielsweise in Gestalt einer Schlaucholive, in einer Vergussmasse eingebettet. Die röhrchenförmigen Endstücke mit den darin eingebetteten Kapillarmembranen, die gleichzeitig die Einlasseinrichtung bzw. Auslasseinrichtung darstellen, lassen sich auf einfache Weise in die Seitenwand des Innenraums des erfindungsgemäßen Membranmoduls fluiddicht beispielsweise mit einem geeigneten Kleber einkleben und mit ihrem freien Ende an eine Zu- bzw. Ableitung anschließen.

Bei der Anwendung des erfindungsgemäßen Membranmoduls ist es vielfach erforderlich, die sich im Zellkulturraum befindlichen Zellen visuell zu beobachten, d.h. beispielsweise zu mikroskopieren. Hierzu sind bei einem bevorzugten Membranmodul Deckel und Boden zueinander parallel angeordnet und jeweils einstückig aus einem transparenten Material ausgebildet.

Vorzugsweise ist der erfindungsgemäße Membranmodul zumindest teilweise mit einer Heizfolie versehen, um eine Temperierung des Innenraums z.B. auf eine Temperatur von 37°C zu ermöglichen. Eine solche Temperierung ist jedoch auch über ein in den Innenraum eingebrachtes Wärmetauschersystem möglich, etwa in Gestalt eines Systems von Kapillaren ähnlich den im Innenraum angeordneten Systemen von Kapillarmembranen, wobei die Kapillaren jedoch flüssigkeitsdichte Wände aufweisen und lumenseitig von einer Temperierflüssigkeit durchströmt werden.

Der erfindungsgemäße Membranmodul ist sowohl für die Wirkstofftestung an Suspensionszellen, d.h. an Zellen, die in Suspension vorliegen, als auch an anhaftungsbedürftigen Zellen geeignet. Anhaftungsbedürftige Zellen können dabei an den im Innenraum des Membranmoduls vorhandenen Oberflächen wie z.B. Seitenwand, Deckel oder Boden oder auch an den im Innenraum angeordneten Kapillarmembranen anhaften oder immobilisiert sein. Jedoch kann es vorkommen, dass bei Anhaftung der Zellen an den Kapillarmembranoberflächen diese nicht mehr vollständig für einen ungehinderten Stoffaustausch zur Verfügung stehen. Der erfindungsgemäße Membranmodul enthält daher in einer bevorzugten Ausgestaltung im Innenraum angeordnete Mittel zur begünstigten Immobilisierung von Zellen, wobei diese Mittel besonders bevorzugt aus textilen Einzelfäden, Geweben oder Gewirken bestehen. Besonders gute Erfahrungen wurden mit Mitteln zur Immobilisierung gemacht, die aus Polyester bestehen. Derartige Mittel werden beispielsweise in der DE-A-36 33 891 beschrieben.

Für Anwendungen, bei denen Suspensionszellen zum Einsatz gelangen, enthält der erfindungsgemäße Membranmodul in einer bevorzugten Ausführungsform schichtförmige Trägermittel, die über die Höhe des Innenraums verteilt zwischen die Schichten der Kapillarmembranen eingebracht sind. Durch diese schichtförmigen Trägermittel wird auch in Fällen, bei denen die Suspensionszellen stark zum Aussedimentieren neigen, eine bessere Verteilung der Suspensionszellen über der Höhe des Zellkulturraums erreicht. Die Trägermaterialien können beispielsweise in Form einer mikroporösen Flachmembran oder in Form eines Vlieses vorliegen, wobei die Porengrößen der Flachmembran bzw. des Vlieses so bemessen sein sollten, dass die Suspensionszellen zumindest weitgehend durch solch eine Flachmembran bzw. durch solch ein Vlies zurückgehalten werden. Durch das Einbringen derartiger Trägermittel werden die Suspensionszellen in verschiedenen Schichten gehalten. Der erfindungsgemäße Membranmodul erlaubt in diesen Fällen zweckmäßgerweise die Einbringung der Suspensionszellen, d.h. das Animpfen mit den Zellen an verschiedenen Positionen über der Höhe des Innenraums, beispielsweise durch eine entsprechende Anzahl Septen, die über die Höhe verteilt in der Seitenwand angeordnet sind. Entsprechendes gilt auch bezüglich der Mittel zur Zuführung von Wirkstoffen.

Aufgrund der erfindungsgemäßen Ausgestaltung herrschen bei der späteren Anwendung im Innenraum des erfindungsgemäßen Membranmoduls im gesamten Innenraum sehr gleichmäßige Bedingungen z.B. hinsichtlich der Nährmediumkonzentration, der Konzentrationen einzelner Gaskomponenten oder auch hinsichtlich der Temperatur. Daher enthält eine bevorzugte Ausführungsform des erfindungsgemäßen Moduls mindestens einen Sensor zur Überwachung der Bedingungen im Innenraum des Moduls, besonders bevorzugt mindestens einen Mikrosensor, d.h. einen Sensor, der hinsichtlich seiner Abmessungen an die kleinen Dimensionen des erfindungsgemäßen Membranmoduls angepasst ist, wobei hinsichtlich der Position solcher Sensoren wegen der gleichmäßigen Bedingungen im Innenraum keine besonderen Anforderungen zu stellen sind. Mittels derartiger Mikrosensoren können beispielsweise der pH-Wert, der Sauerstoffpartialdruck, Glukosekonzentrabon, Laktatkonzentration, Druck und/oder Temperatur im Innenraum on-line gemessen werden, wobei es durch die Miniaturisierung zu keinem störenden Einfluss des Sensors bzw. der Sensoren auf die im Zellkulturraum befindliche Zellkultur kommt.

Die Erfindung wird nachfolgend anhand der Figuren näher erläutert. Es zeigen in vereinfachter schematischer Darstellung:
- Fig. 1:: Querschnitt durch einen erfindungsgemäßen Membranmodul
- Fig. 2:: Draufsicht auf den in Fig. 1 dargestellten erfindungsgemäßen Membranmodul mit teilweisem Ausbruch zur Darstellung der Verteilung der Kapillarmembranen im Innenraum

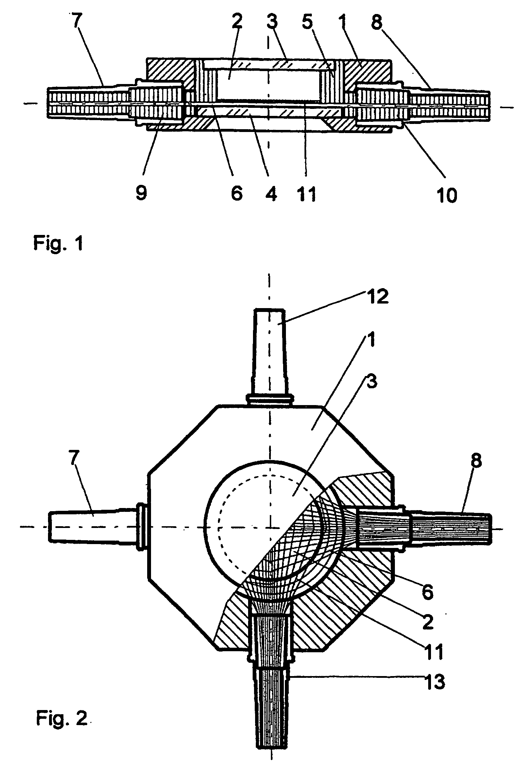

In Figur 1 ist in vergrößertem Maßstab ein erfindungsgemäßer Membranmodul dargestellt, welcher ein mehrteiliges Gehäuse 1 mit einem Innenraum 2 zur Aufnahme einer Zellkultur aufweist. Der Innenraum 2 wird von einem Deckel 3, einem zum Deckel parallelen Boden 4 und einer Seitenwand 5 begrenzt und weist in Blickrichtung senkrecht zum Deckel bzw. Boden einen kreisförmigen innenquerschnitt auf. Deckel 3 und Boden 4 bestehen aus transparentem Material, um eine Beobachtung der Zellen im Innenraum zu ermöglichen. Die Seitenwand 5 besteht im vorliegenden Beispiel aus einer ausgehärteten Vergussmasse, in die gleichzeitig die sich in Bodennähe befindlichen Kapillarmembranen eingebettet sind, so dass ein fluiddichter Abschluss zwischen Innenraum 2 und Aussenseite des Gehäuses resultiert.

Im Innenraum sind erste Kapillarmembranen 6 in Form einer einzelnen Schicht angeordnet, die im cross flow Modus durchströmbar sind und deren Enden auf gegenüberliegenden Seiten durch die Seitenwand des Innenraums aus dem Innenraum austreten und in Schlaucholiven 7,8 jeweils in eine Vergussmasse 9,10 eingebettet sind. Der Membranmodul gemäß Fig. 1 enthält des Weiteren eine Schicht zweite Kapillarmembranen 11, die in der vorliegenden Darstellung senkrecht zur Zeichnungsebene verlaufen.

Figur 2 zeigt den in Figur 1 dargestellten Membranmodul in der Draufsicht mit einem teilweisen Ausbruch, um die Anordnung der Kapillarmembranen im Innenraum des Moduls zu verdeutlichen. Der Innenraum 2 wird vom Gehäuse 1 umschlossen und mit einem kreisförmigen Deckel 3 abgedeckt. Im Innenraum 2 sind die ersten und die zweiten Kapillarmembranen 6,11 gleichmäßig über den Innenquerschnitt des Innenraums 2 verteilt. Die ersten Kapillarmembranen 6 verlaufen in dieser Darstellung von rechts nach links, wobei sie zur gleichmäßigen Verteilung über den Innenquerschnitt im Innenraum aufgefächert sind und wobei ihre Enden in den Schlaucholiven 7,8 zu Bündeln zusammengefasst und dort eingebettet sind. Die zweiten Kapillarmembranen 11 verlaufen in dieser Darstellung von oben nach unten, sind ebenfalls zur gleichmäßigen Verteilung über den Innenquerschnitt im Innenraum aufgefächert, an ihren Enden zu Bündeln zusammengefasst und in den Schlaucholiven 12,13 eingebettet. Im Innenraum 2 überkreuzen sich die ersten und zweiten Kapillarmembranen 6,11.

## Patentansprüche

1. Membranmodul zum Testen von Wirkstoffen an Zellen, umfassend ein Gehäuse (1) mit einem Innenraum (2), welcher durch einen Deckel (3), einen Boden (4) sowie eine Seitenwand (5) begrenzt wird, im Innenraum (2) angeordnet ein System von ersten Kapillarmembranen (6) und ein System von zweiten Kapillarmembranen (11) und gegebenenfalls mindestens ein weiteres System von Kapillarmembranen, wobei jede Kapillarmembran jeweils ein erstes und ein zweites Ende sowie ein mit einem Fluid beschickbares Lumen aufweist, wobei die Kapillarmembranen im innenraum (2) zu mindestens einer zum Boden (4) parallelen flächigen Schicht angeordnet sind und im Innenraum (2) des Gehäuses (1) im extrakapillären Raum um die Kapillarmembranen herum ein Zellkulturraum ausgebildet ist, wobei die Kapillarmembranen mit mindestens einem ihrer Enden durch die Seitenwand (5) des Innenraums (2) hindurchtreten und so nach Systemen getrennt an diesem mindestens einen Ende jeweils in einer Vergussmasse (9,10) eingebettet sind, dass der Innenraum (2) fluiddicht nach außen abgedichtet ist, und wobei die Kapillarmembranen eines jeden Systems mit ihrem Lumen mit einer Einlasseinrichtung (7,12) und/oder einer Auslasseinrichtung (8,13) in Fluidverbindung stehen, **dadurch gekennzeichnet, dass** der Innenraum (2) ein Volumen zwischen 0,1 und 5 cm³ aufweist.

2. Membranmodul nach Anspruch 1, **dadurch gekennzeichnet, dass** der Innenraum (2) in Blickrichtung senkrecht zur mindestens einen Schicht einen kreisförmigen Innenquerschnitt aufweist.

3. Membranmodul nach einem der Ansprüche 1 oder 2, **dadurch gekennzeichnet, dass** der Innenraum (2) einen Durchmesser zwischen 10 und 20 mm aufweist.

4. Membranmodul nach einem oder mehreren der Ansprüche 1 bis 3, **dadurch gekennzeichnet, dass** Deckel (3) und Boden (4) zueinander parallel angeordnet und jeweils einstückig aus transparentem Material ausgebildet sind.

5. Membranmodul nach einem oder mehreren der Ansprüche 1 bis 4, **dadurch gekennzeichnet, dass** die Kapillarmembranen in Blickrichtung senkrecht zur mindestens einen Schicht innerhalb einer Schicht gleichmäßig über den Innenquerschnitt des Innenraums (2) verteilt sind.

6. Membranmodul nach einem oder mehreren der Ansprüche 1 bis 5, **dadurch gekennzeichnet, dass** die Kapillarmembranen unterschiedlicher Systeme in eine in Blickrichtung senkrecht zur mindestens einen Schicht sich überkreuzende Anordnung gebracht sind.

7. Membranmodul nach einem oder mehreren der Ansprüche 1 bis 6, **dadurch gekennzeichnet, dass** er Mittel zur Zuführung eines Wirkstoffes in den Innenraum umfasst.

8. Membranmodul nach Anspruch 7, **dadurch gekennzeichnet, dass** die Mittel zur Zuführung eines Wirkstoffes aus mindestens einem Einlass in der Seitenwand (5) bestehen.

9. Membranmodul nach Anspruch 7, **dadurch gekennzeichnet, dass** die Mittel aus einem weiteren System von Kapillarmembranen bestehen.

10. Membranmodul nach einem oder mehreren der Ansprüche 1 bis 9, **dadurch gekennzeichnet, dass** die Enden der Kapillarmembranen der einzelnen Systeme jeweils zu Bündeln zusammengefasst und in ein röhrchenförmiges Endstück eingebettet sind.

11. Membranmodul nach einem oder mehreren der Ansprüche 1 bis 10, **dadurch gekennzeichnet, dass** im Innenraum mehrere Schichten von Kapillaren angeordnet sind, die über der Höhe des Innenraums (2) zwischen Deckel (3) und Boden (4) asymmetrisch mit einer dichteren Schichtenfolge in der Nähe des Bodens (4) verteilt sind.

12. Membranmodul nach einem oder mehreren der Ansprüche 1 bis 11, **dadurch gekennzeichnet, dass** im Innenraum (2) Mittel zur begünstigten Immobilisierung von Zellen angeordnet sind.

13. Membranmodul nach Anspruch 12, **dadurch gekennzeichnet, dass** die Mittel aus textilen Einzelfäden, Geweben oder Gewirken bestehen.

14. Membranmodul nach einem oder mehreren der Ansprüche 1 bis 13, **dadurch gekennzeichnet, dass** die ersten Kapillarmembranen (6) Mikrofiltrationsmembranen sind, die zur Zuführung eines flüssigen Nährmediums in den Innenraum geeignet sind, und die zweiten Kapillarmembranen (11) Membranen sind, die zur Zuführung eines gasförmigen Stoffes in den Innenraum geeignet sind.

15. Verwendung des Membranmoduls nach einem oder mehreren der Ansprüche 1 bis 14 zum Testen von Wirkstoffen an Zellen.

## Claims

1. A membrane module for testing active substances on cells, comprising a housing (1) with an interior chamber (2), bounded by a lid (3), a base (4), and a side wall (5); arranged in the interior chamber (2), a system of first capillary membranes (6) and a system of second capillary membranes (11), and optionally one or more additional systems of capillary membranes, wherein each capillary membrane has a first and a second end as well as a lumen through which a fluid can be transported, wherein the capillary membranes in the interior chamber (2) are arranged in at least one flat layer parallel to the base (4), and in the interior chamber (2) of the housing (1), in an extracapillary space around the capillary membranes, a cell culture chamber is formed, wherein at least one end of each of the capillary membranes penetrates through the side wall (5) of the interior chamber (2), and the capillary membranes, grouped by system, are embedded at these ends in a sealing compound (9,10) such that the interior chamber (2) is sealed off in a fluid-tight manner from the exterior, and wherein the lumens of the capillary membranes of each system are in fluid communication with an inlet device (7,12), or an outlet device (8,13), or both, **characterised in that** the interior chamber (2) has a volume between 0.1 and 5 cm³.

2. Membrane module according to Claim 1, **characterised in that** the interior chamber (2), viewed in a direction perpendicular to the at least one layer, has a circular interior cross section.

3. Membrane module according to Claim 1 or 2, **characterised in that** the interior chamber (2) has a diameter between 10 and 20 mm.

4. Membrane module according to one or more of Claims 1 to 3, **characterised in that** the lid (3) and the base (4) are parallel to each other, each being made from a single piece of transparent material.

5. Membrane module according to one or more of Claims 1 to 4, **characterised in that** the capillary membranes, viewed in a direction perpendicular to the at least one layer, are uniformly distributed over the internal cross section of the interior chamber (2) within a layer.

6. Membrane module according to one or more of Claims 1 to 5, **characterised in that** the capillary membranes of different systems, viewed in a direction perpendicular to the at least one layer, are arranged in a crosswise manner.

7. Membrane module according to one or more of Claims 1 to 6, **characterised in that** the membrane module comprises means for supplying an active substance to the interior chamber.

8. Membrane module according to Claim 7, **characterised in that** the means for supplying an active substance consist of at least one inlet in the side wall (5).

9. Membrane module according to Claim 7, **characterised in that** the means consist of a further system of capillary membranes.

10. Membrane module according to one or more of Claims 1 to 9, **characterised in that** the ends of the capillary membranes of the individual systems are collected into bundles and are embedded in a tubular end-piece.

11. Membrane module according to one or more of Claims 1 to 10, **characterised in that** a plurality of layers of capillaries are located in the interior chamber, which are distributed asymmetrically over the height of the interior chamber (2) between the lid (3) and the base (4), with a higher density of layers in the vicinity of the base (4).

12. Membrane module according to one or more of Claims 1 to 11, **characterised in that** means for facilitating immobilisation of cells are located in the interior chamber (2).

13. Membrane module according to Claim 12, **characterised in that** the means consist of single textile filaments, woven fabrics or knitted fabrics.

14. Membrane module according to one or more of Claims 1 to 13, **characterised in that** the first capillary membranes (6) are microfiltration membranes suitable for supply of a liquid nutrient medium to the interior chamber, and that the second capillary membranes (11) are membranes suitable for supply of a gaseous substance to the interior chamber.

15. The use of the membrane module according to one or more of Claims 1 to 14 for testing of active substances on cells.

## Revendications

1. Module à membranes servant à tester des matières actives sur des cellules, ce module à membranes comprenant un boîtier (1) avec un espace intérieur (2) délimité par un couvercle (3), un fond (4) ainsi qu'une paroi latérale (5), un système de premières membranes capillaires (6) et un système de deuxièmes membranes capillaires (11) étant disposés dans l'espace intérieur (2) ainsi que, le cas échéant, au moins un autre système de membranes capillaires (6), chaque membrane capillaire comportant une première extrémité et une deuxième extrémité respectives ainsi qu'un lumen qui peut être chargé d'un fluide, alors que les membranes capillaires dans l'espace intérieur (2) sont disposées par rapport à au moins une couche plane à deux dimensions parallèle au fond (4) et que, dans l'espace intérieur (2) du boîtier (1), se trouve un espace de culture de cellules configuré dans l'espace extra-capillaire tout autour des membranes capillaires, ces membranes capillaires passant avec au moins l'une de leurs extrémités à travers la paroi latérale (5) de l'espace intérieur (2) de sorte que, tout en étant séparées suivant les systèmes, elles se trouvent de cette façon noyées par cette extrémité au moins présente, dans une masse de scellement (9, 10) respective, alors que l'espace intérieur (2) est isolé vers l'extérieur d'une manière étanche aux fluides, et que les membranes capillaires de chacun des systèmes se trouvent avec leur lumen en communication de fluide avec une installation d'entrée (7, 12) et / ou avec une installation de sortie (8, 13), **caractérisé en ce que** l'espace intérieur (2) possède un volume compris entre 0,1 et 5 cm³.

2. Module à membranes suivant la revendication 1, **caractérisé en ce que** l'espace intérieur (2) présente une section transversale interne ronde qui, vue dans la direction de visée, est perpendiculaire par rapport à la couche au moins présente.

3. Module à membranes suivant la revendication 1 ou 2, **caractérisé en ce que** l'espace intérieur (2) présente un diamètre compris entre 10 et 20 mm.

4. Module à membranes suivant l'une ou plusieurs des revendications 1 à 3, **caractérisé en ce que** le couvercle (3) et le fond (4) sont disposés de manière parallèle l'un par rapport à l'autre, et sont chacun façonnés en une seule pièce à partir d'un matériau transparent.

5. Module à membranes suivant l'une ou plusieurs des revendications 1 à 4, **caractérisé en ce que** les membranes capillaires sont, dans la direction du regard, réparties perpendiculairement par rapport à couche au moins présente et, à l'intérieur d'une couche, de façon uniforme sur la section transversale interne de l'espace intérieur (2).

6. Module à membranes suivant l'une ou plusieurs des revendications 1 à 5, **caractérisé en ce que** les membranes capillaires de différents systèmes sont aménagées, dans la direction du regard, perpendiculaires par rapport à la couche au moins présente, dans une disposition en forme de croix.

7. Module à membranes suivant l'une ou plusieurs des revendications 1 à 6, **caractérisé en ce qu'**il comporte des moyens pour l'introduction d'une matière active dans l'espace intérieur.

8. Module à membranes suivant la revendication 7, **caractérisé en ce que** les moyens pour l'introduction d'une matière active se composent d'au moins une admission dans la paroi latérale (5).

9. Module à membranes suivant la revendication 7, **caractérisé en ce que** les moyens se composent d'un autre système complémentaire de membranes capillaires.

10. Module à membranes suivant l'une ou plusieurs des revendications 1 à 9, **caractérisé en ce que** les extrémités des membranes capillaires des divers systèmes individuels respectifs sont rassemblées en des faisceaux, et sont noyées dans une pièce terminale en forme de tube.

11. Module à membranes suivant l'une ou plusieurs des revendications 1 à 10 **caractérisé en ce que**, dans l'espace intérieur, sont aménagées plusieurs couches de capillaires qui sont réparties sur la hauteur de l'espace intérieur (2), entre le couvercle (3) et le fond (4), d'une manière asymétrique, une suite plus dense de couches se trouvant à proximité du fond (4).

12. Module à membranes suivant l'une ou plusieurs des revendications 1 à 11, **caractérisé en ce que**, dans l'espace intérieur (2) sont disposés des moyens servant à une immobilisation favorisée de cellules.

13. Module à membranes suivant la revendication 12, **caractérisé en ce que** les moyens sont constitués de fils textiles individuels, de structures tissées ou de structures tricotées.

14. Module à membranes suivant l'une ou plusieurs des revendications 1 à 13, **caractérisé en ce que** les premières membranes capillaires (6) sont constituées de membranes destinées à la microfiltration, qui sont appropriées pour l'amenée de milieux de culture liquides dans l'espace intérieur, et **en ce que** les deuxièmes membranes capillaires (11) sont constituées de membranes qui sont appropriées pour l'amenée d'un produit gazeux dans l'espace intérieur.

15. Utilisation du module à membranes suivant l'une ou plusieurs des revendications 1 à 14, pour tester des matières actives sur des cellules.
